# EUROPÄISCHE PATENTSCHRIFT

(11) **EP 3 968 850 B1**
(45) Veröffentlichungstag und Bekanntmachung des Hinweises auf die Patenterteilung: **07.08.2024**
(21) Anmeldenummer: 20733250.3
(22) Anmeldetag: 12.06.2020
(51) Int. Cl.: G01L 1/14, A61B 5/11, G01L 1/24, G01K 11/32, B60N 2/00, A61B 5/00, A61B 5/18, A61B 5/16, A61B 5/024, A61B 5/021, A61B 5/01

(54) **SYSTEM ZUM ERFASSEN VON BIOSIGNALEN EINER PERSON, FORTBEWEGUNGSMITTEL MIT EINEM SOLCHEN SYSTEM**
SYSTEM FOR DETECTING BIOSIGNALS OF A PERSON, MEANS OF TRANSPORT COMPRISING SUCH A SYSTEM
SYSTÈME DE DÉTECTION DES BIOSIGNAUX D'UNE PERSONNE, MOYENS DE TRANSPORT COMPRENANT UN TEL SYSTÈME

(30) Priorität: 22.07.2019 DE 102019210796
(43) Veröffentlichungstag der Anmeldung: 23.03.2022
(73) Patentinhaber: VOLKSWAGEN AKTIENGESELLSCHAFT, 38440 Wolfsburg (DE)
(72) Erfinder: ZLATKOV, Branislav, 38104 Braunschweig (DE); QUANDER, Nils, 38154 Königslutter (DE); BOESE, Christoph, 38302 Wolfenbüttel (DE)
(86) Internationale Anmeldenummer: PCT/EP2020/066346
(87) Internationale Veröffentlichungsnummer: WO 2021/013431

(56) Entgegenhaltungen:
- WO-A1-2015/138416
- CN-A- 108 567 433
- DZIUDA L ET AL: "Monitoring Respiration and Cardiac Activity Using Fiber Bragg Grating-Based Sensor", IEEE TRANSACTIONS ON BIOMEDICAL ENGINEERING, IEEE SERVICE CENTER, PISCATAWAY, NJ, USA, vol. 59, no. 7, 1 July 2012 (2012-07-01), pages 1934 - 1942, XP011490124, ISSN: 0018-9294, DOI: 10.1109/TBME.2012.2194145
- BRIT M. QUANDT ET AL: "Body-Monitoring and Health Supervision by Means of Optical Fiber-Based Sensing Systems in Medical Textiles", ADVANCED HEALTHCARE MATERIALS, vol. 4, no. 3, 1 February 2015 (2015-02-01), DE, pages 330 - 355, XP055719648, ISSN: 2192-2640, DOI: 10.1002/adhm.201400463
- SULEIMAN M ET AL: "Interrogation of Fiber Bragg Grating Dynamic Strain Sensors by Self-Mixing Interferometry", IEEE SENSORS JOURNAL, IEEE SERVICE CENTER, NEW YORK, NY, US, vol. 8, no. 7, 1 July 2008 (2008-07-01), pages 1317 - 1323, XP011231378, ISSN: 1530-437X, DOI: 10.1109/JSEN.2008.926961
- IAN YULIANTI ET AL: "Design of fiber Bragg grating-based Fabry-Perot sensor for simultaneous measurement of humidity and temperature", OPTIK., vol. 124, no. 19, 1 October 2013 (2013-10-01), DE, pages 3919 - 3923, XP055719530, ISSN: 0030-4026, DOI: 10.1016/j.ijleo.2012.11.043

## Beschreibung

Die vorliegende Erfindung betrifft ein System zum Erfassen von Biosignalen einer Person. Die Erfindung betrifft weiterhin ein Fortbewegungsmittel, insbesondere ein Kraftfahrzeug, in dem ein solches System genutzt wird.

Im Zuge der zunehmenden Ausstattung von Fortbewegungsmitteln mit Assistenzsystemen, die den Bediener unterstützen und entlasten sollen, wird vermehrt versucht, den Gesundheitszustand des Bedieners bzw. der Insassen zu überwachen, beispielsweise um im Falle von drohenden Gefahrensituationen geeignete Maßnahmen einzuleiten.

In diesem Zusammenhang beschreibt DE 10 2011 113 100 A1 ein Verfahren zur Detektion ballistokardiogener oder atmungsbedingter Bewegungen einer auf einem Fahrzeugsitz eines Kraftfahrzeugs sich befindenden Person. Bei dem Verfahren ist ein erster Sensor als ballistographischer Sensor zur Erfassung der ballistokardiogenen oder atmungsbedingten Bewegungen der Person ausgebildet und liefert ein ballistographisches Signal. Ein zweiter Sensor erzeugt ein Schwingungssignal als Störreferenz für das ballistographische Signal.

DE 10 2014 003 783 A1 beschreibt eine Sicherheitseinrichtung für ein Kraftfahrzeug, mit wenigstens einem Sensor zum Überwachen wenigstens einer Vitalfunktion eines Fahrers. Der Sensor kann ein Herzfrequenzmessgerät im Fahrzeugsitz umfassen. Eine Auswerteeinheit wertet Sensordaten aus und ist dazu ausgebildet, eine Beeinträchtigung des Fahrers zu erkennen, insbesondere einen Schlaganfall des Fahrers zu diagnostizieren. Die Sicherheitseinrichtung ist dazu ausgebildet, das Kraftfahrzeug bei einer erkannten Beeinträchtigung in einen sicheren Zustand zu überführen.

DE 10 2014 216 397 A1 beschreibt einen Sensor zur berührungslosen elektrokardiographischen Messung an einer Person. Der Sensor kann in einem Fahrzeugsitz angeordnet sein und umfasst ein flächiges Trägerelement zur Befestigung des Sensors an einem Objekt und wenigstens eine elektrisch leitfähige, flächige, dem Trägerelement gegenüberliegende und mit diesem verbundene Elektrode. Ferner stützt sich die Elektrode über wenigstens einen Drucksensor an dem Trägerelement ab. Zwischen dem Trägerelement und der Elektrode sind außerdem ein Feuchtesensor, ein Temperatursensor und ein Beschleunigungssensor angeordnet.

DE 10 2016 222 693 A1 beschreibt ein Messsystem für ein biologisches Signal für einen Fahrzeugsitz. Das Messsystem umfasst eine Messvorrichtung für ein biologisches Signal, die eine elektrostatische kapazitive Kopplung nutzt. Die Messvorrichtung ist in einem Sitz eines Fahrzeugs vorgesehen, in welchem sich der größte Teil des Körpers des Fahrers, welcher ein Fahrzeug fährt, beständig befindet. Das Messsystem ist dazu ausgebildet, ein Störsignal von dem biologischen Signal zu bestimmen und zu entfernen, welches durch die Bewegung des Fahrers aufgrund einer Veränderung einer Fahrumgebung und eines Straßenzustands verursacht wird.

DE 10 2017 211 305 A1 beschreibt einen Fahrzeugsitz mit einer Vorrichtung zur Detektion biologischer Information, z.B. einer Herzschlagfrequenz oder einer Atemfrequenz. Die Vorrichtung verwendet einen Sender, welcher eine Radiowelle in Richtung eines Körperteils aussendet, und einen Empfänger, welcher eine von einem Körperabschnitt reflektierte Welle empfängt.

Zwar finden sich im Stand der Technik zahlreiche partielle Lösungen, um einzelne Biosignale von Personen zu erfassen, allerdings fehlt ein umfassendes Konzept, wie mit einem vertretbaren sensorischen Aufwand eine Vielzahl verschiedener Biosignale erfasst werden kann.

In diesem Kontext beschreibt der Artikel L. Dziuda et al.: "Monitoring Respiration and Cardiac Activity Using Fiber Bragg Grating-Based Sensor", IEEE Transactions on Biomedical Engineering, Vol. 59 (2012), S. 1934-1942, einen faseroptischen Sensor zur Erfassung von Atmung und Herzschlag einer auf einem Sitz befindlichen Person. Der faseroptische Sensor nutzt eine Monomodefaser, in die ein Bragg-Gitter eingeschrieben ist. Ein auf das Bragg-Gitter ausgeübter Druck führt zu einer Änderung der Bragg-Wellenlänge. Die zeitliche Entwicklung der der Bragg-Wellenlänge wird mit einem Fabry-Pérot-Filter bestimmt. Aus dem zeitlichen Verlauf werden dann Atem- und Herzfrequenz ermittelt.

CN 108567433 A beschreibt eine multifunktionale, rein faseroptische, nicht-intrusive, dünne Matte zur Zustandsüberwachung. Die Matte umfasst eine erste Mattenschicht, eine zweite Mattenschicht und ein Zustandsüberwachungssystem. Das Zustandsüberwachungssystem umfasst unter anderem einen Zustandssensor und einen photoelektrischen Wandler. Der Zustandssensor ist zwischen der ersten Mattenschicht und der zweiten Mattenschicht angeordnet, eine erste optische Faser ist zwischen einer Lichtquelle und einem Eingangsende des Zustandssensors angeordnet, und eine zweite optische Faser ist zwischen einem Ausgangsende des Zustandssensors und dem photoelektrischen Wandler angeordnet.

Es ist eine Aufgabe der Erfindung, ein verbessertes System zum Erfassen von Biosignalen einer Person bereitzustellen.

Diese Aufgabe wird durch ein System mit den Merkmalen des Anspruchs 1 und durch ein Fortbewegungsmittel gemäß Anspruch 8 gelöst. Bevorzugte Ausgestaltungen der Erfindung sind Gegenstand der abhängigen Ansprüche.

Gemäß einem Aspekt der Erfindung umfasst ein System zum Erfassen von Biosignalen einer Person eine biometrische Sitzvorrichtung für die Person, wobei die biometrische Sitzvorrichtung zumindest ein auf optischer Interferometrie basierendes faseroptisches Sensorelement aufweist, das als Michelson-Interferometer ausgestaltet ist und eine Lichtquelle aufweist, und eine Auswerteeinheit zum Auswerten von Signalen des zumindest einen faseroptischen Sensorelementes. Das System ist eingerichtet, auf Grundlage der Signale des zumindest einen faseroptischen Sensorelementes Biosignale der Person zu ermitteln. Das faseroptische Sensorelement weist einen Faserkoppler und zwei Photodioden auf. Die Lichtquelle ist mit einem mittleren Arm des Faserkopplers verbunden, der mit einem optischen Isolator versehen ist. Das Licht der Lichtquelle wird durch den Faserkoppler zumindest in einen Sensorarm und einen Referenzarm eingekoppelt. Das eingekoppelte Licht wird an den Faserenden reflektiert, läuft wieder durch den Faserkoppler zurück, interferiert dabei und wird in zwei Eingangsarme aufgespalten. Dort erfolgt eine Detektion mittels der zwei Photodioden.

Während des Betriebs eines Fortbewegungsmittels oder bei der Nutzung von Fahrgeschäften oder ähnlichen Einrichtungen sitzen die Insassen die überwiegende Zeit in einer Sitzvorrichtung. Es ist daher sinnvoll, die Sitzvorrichtung mit zumindest einem Sensorelement zum Erfassen von Biosignalen zu versehen. Unter einer Sitzvorrichtung ist dabei ein Sitz mit einer Sitzfläche und gegebenenfalls einer Rückenlehne und einer Kopfstütze zu verstehen. Zusätzlich kann die Sitzvorrichtung auch zumindest eine Armlehne sowie einen Sicherheitsgurt aufweisen.

Die Verwendung eines auf optischer Interferometrie basierenden faseroptischen Sensorelementes hat den Vorteil, dass sehr genaue Messungen möglich sind und die Messtechnik eine hohe elektromagnetische Verträglichkeit aufweist. Dieses Messprinzip ist hochempfindlich und erlaubt es, selbst sehr kleine Messgrößen mit hoher Genauigkeit zu detektieren. Gleichzeitig kann mittels optischer Messungen eine Vielzahl verschiedener Biosignale ermitteln werden. Beispielsweise können die Temperatur, der Herzschlag, die Herzfrequenzvariabilität, der Anpressdruck an eine Rückenlehne, der Blutdruck, die Schweißbildung oder die Masse der Person und deren Verteilung auf einer Sitzfläche der biometrischen Sitzvorrichtung ermittelt werden. Selbstverständlich können dazu auch mehrere, auf die jeweilige Messgröße angepasste faseroptische Sensorelemente verwendet werden.

Die ermittelten Größen können im Rahmen einer Analyse genutzt werden, um ein Phonokardiogramm zu erstellen oder eine Kardioanalyse, eine Bestimmung einer Stimmungslage oder eine psychophysische Zustandsüberwachung vorzunehmen. Dabei kann die Analyse die Ergebnisse in Bezug setzen zu verfügbaren Informationen, beispielsweise zur Verkehrssituation (Stau, Stop-and-go, etc.), zum Fahrverhalten (Dynamik des Beschleunigens oder Bremsens, aggressive Richtungsänderungen, angepasste oder nichtangepasste Geschwindigkeit, etc.) oder zu den Wetterumständen (Regen, Nebel, Böen, etc.). Im Rahmen der psychophysischen Zustandsüberwachung kann ermittelt werden, ob der Bediener Anzeichen von Müdigkeit, Stress, Konzentrationsschwäche oder Nervosität zeigt, ob seine Reaktionen vermindert sind oder wie es allgemein um sein Wohlbefinden steht. Auch Rückschlüsse auf Ängste oder Panikattacken, z.B. durch einen Unfall oder eine Gefahrensituation, sind möglich.

Bei Bedarf können der beobachteten Person in Abhängigkeit vom Ergebnis der psychophysischen Zustandsüberwachung Maßnahmen vorgeschlagen werden, z.B. Bewegungs- oder Dehnübungen bei Müdigkeit. Ebenso ist es möglich, im Falle von Notsituationen Sicherheitsmanöver einzuleiten, um das Fortbewegungsmittel in einen sicheren Zustand zu bringen. Im Falle eines Unglückes, z.B. durch einen Verkehrsunfall, kann der Nothelfer oder Notarzt wichtige Werte, wie die Blutdruckwerte, den Puls, den Atemverlauf etc., direkt übernehmen, ohne die verunglückte Person bewegen zu müssen. Dazu kann beispielsweise eine Bluetooth-Verbindung mit der Auswerteeinheit hergestellt werden. Voraussetzung ist lediglich, dass die Sensortechnik trotz des Unfalls noch funktionsfähig ist. Die Möglichkeit der Übernahme der Werte durch einen Nothelfer oder Notarzt ist insbesondere dann von Vorteil, wenn die betroffene Person stark verletzt oder ohne Bewusstsein ist.

Die Verwendung eines faseroptischen Sensorelementes hat zudem den Vorteil, dass es einen ausgedehnten Bereich der Sitzvorrichtung abdecken kann, so dass die Messungen nicht auf einzelne aktive Messpunkte beschränkt sind. Dabei sind unterschiedliche Formen des Verlaufs der Faser möglich. Beispielsweise kann die Faser mäanderförmig oder im Zick-Zack angeordnet werden. Ebenso kann eine ringförmige oder eine spiralförmige Verlegung erfolgen. Weitere Verlegeformen sind dem Fachmann bekannt.

Gemäß einem Aspekt der Erfindung weist das faseroptische Sensorelement zumindest eine Monomodefaser auf. Die Verwendung einer Monomodefaser hat den Vorteil, dass eine sehr hohe Auflösung und damit eine sehr genaue Messung ermöglicht wird. Optische Interferometrie funktioniert zudem besonders gut in Verbindung mit einer Monomodefaser. Monomodefasern können aus unterschiedlichen Materialen hergestellt werden, z.B. aus Glas oder einer Glasmischung, Quarz, einem Elastomer oder einem Thermoplast wie Polycarbonat, Polymethylmethacrylat, Polystyrol, etc.

Gemäß einem Aspekt der Erfindung ist die Auswerteeinheit eingerichtet, eine durch eine Kraft hervorgerufene Änderung zumindest einer optischen Eigenschaft des zumindest einen faseroptischen Sensorelementes zu erfassen. Beispielsweise ändert sich durch eine von einer Person auf eine optische Faser ausgeübte Kraft zumindest lokal der Brechungsindex der optischen Faser, was zu einer Laufzeitänderung eines optischen Signals führt, welches sich in der Faser ausbreitet. Da z.B. der Herzschlag der Person als Druckstoß über die Blutbahnen des Bedieners übertragen wird, kann der Herzschlag anhand der Änderung der auf das faseroptische Sensorelement ausgeübten Kraft ausgewertet werden.

Gemäß einem Aspekt der Erfindung ist das zumindest eine faseroptische Sensorelement in einer Sitzfläche, einer Rückenlehne, einer Kopfstütze oder einer Armlehne der biometrischen Sitzvorrichtung angeordnet. Bei der Nutzung einer Sitzvorrichtung durch eine Person befindet sich diese Person fortlaufend in Kontakt mit der Sitzfläche und, sofern vorhanden, der Rückenlehne und der Kopfstütze. Durch die Anordnung des faseroptischen Sensorelementes in der Sitzfläche, der Rückenlehne oder der Kopfstütze ist somit eine kontinuierliche Überwachung der gewünschten Biosignale möglich. Die Anordnung eines faseroptischen Sensorelementes in einer eventuell vorhandenen Armlehne hat darüber hinaus den Vorteil, dass bei Tragen von ärmelloser oder kurzärmeliger Kleidung ein Hautkontakt mit der Armlehne bestehen kann, wodurch genauere Messungen und insbesondere auch die Untersuchung der Schweißbildung ermöglicht werden.

Gemäß einem Aspekt der Erfindung ist das zumindest eine faseroptische Sensorelement in einem Sicherheitsgurt oder einem Sicherheitsbügel der biometrischen Sitzvorrichtung angeordnet. Ein Vorteil dieser Ausführung ist es, dass der Sicherheitsgurt bzw. der Sicherheitsbügel in der Regel zwingend genutzt werden muss. Ein Sicherheitsgurt liegt zudem direkt an der Oberfläche des Körpers an. Trotz der in der Regel zwischen Gurt und Körper befindlichen Kleidung lassen sich so genauere Signale erfassen. Insbesondere im Hals- bzw. Schulterbereich kann zudem ein direkter Hautkontakt mit dem Sicherheitsgurt bestehen, wodurch genauere Messungen und insbesondere auch die Untersuchung der Schweißbildung ermöglicht werden.

Besonders vorteilhaft verwendet ein Fortbewegungsmittel ein erfindungsgemäßes System zum Erfassen von Biosignalen eines Bedieners des Fortbewegungsmittels. Bei dem Fortbewegungsmittel kann es sich insbesondere um ein Kraftfahrzeug handeln, z.B. um einen Personenkraftwagen, ein Nutzfahrzeug oder einen Bus. Daneben ist aber auch eine Verwendung in Schienenfahrzeugen, Flugzeugen, Schiffen, Seilbahnen o.ä. sinnvoll. Zudem kann die erfindungsgemäße Lösung in Fahrgeschäften oder ähnlichen Anlagen zum Einsatz kommen, die Sitzvorrichtungen für die Nutzer bzw. Insassen bereithalten.

Weitere Merkmale der vorliegenden Erfindung werden aus der nachfolgenden Beschreibung und den angehängten Ansprüchen in Verbindung mit den Figuren ersichtlich.
- Fig. 1: zeigt schematisch ein System zum Erfassen von Biosignalen einer Person;
- Fig. 2: zeigt schematisch eine erste Ausführungsform einer Auswerteeinheit, die zur Verwendung im System aus Fig. 1 geeignet ist;
- Fig. 3: zeigt schematisch eine zweite Ausführungsform einer Auswerteeinheit, die zur Verwendung im System aus Fig. 1 geeignet ist;
- Fig. 4: zeigt schematisch ein Verfahren zum Auswerten von Signalen eines optischen Sensors einer biometrischen Sitzvorrichtung;
- Fig. 5: stellt schematisch ein Kraftfahrzeug dar, in dem eine erfindungsgemäßes System realisiert ist;
- Fig. 6: stellt schematisch den Aufbau eines faseroptischen Sensorelementes dar;
- Fig. 7: illustriert das Messprinzip, auf dem das faseroptische Sensorelement basiert;
- Fig. 8: zeigt ein mit dem faseroptischen Sensorelement gemessenes Phonokardiogramm;
- Fig. 9: zeigt ein mit dem faseroptischen Sensorelement gemessenes Herzschlagsignal; und
- Fig. 10: zeigt eine Analyse der Herztöne auf Basis des Herzschlagsignals aus Fig. 9.

Zum besseren Verständnis der Prinzipien der vorliegenden Erfindung werden nachfolgend Ausführungsformen der Erfindung anhand der Figuren detaillierter erläutert. Es versteht sich, dass sich die Erfindung nicht auf diese Ausführungsformen beschränkt und dass die beschriebenen Merkmale auch kombiniert oder modifiziert werden können, ohne den Schutzbereich der Erfindung zu verlassen, wie er in den angehängten Ansprüchen definiert ist.

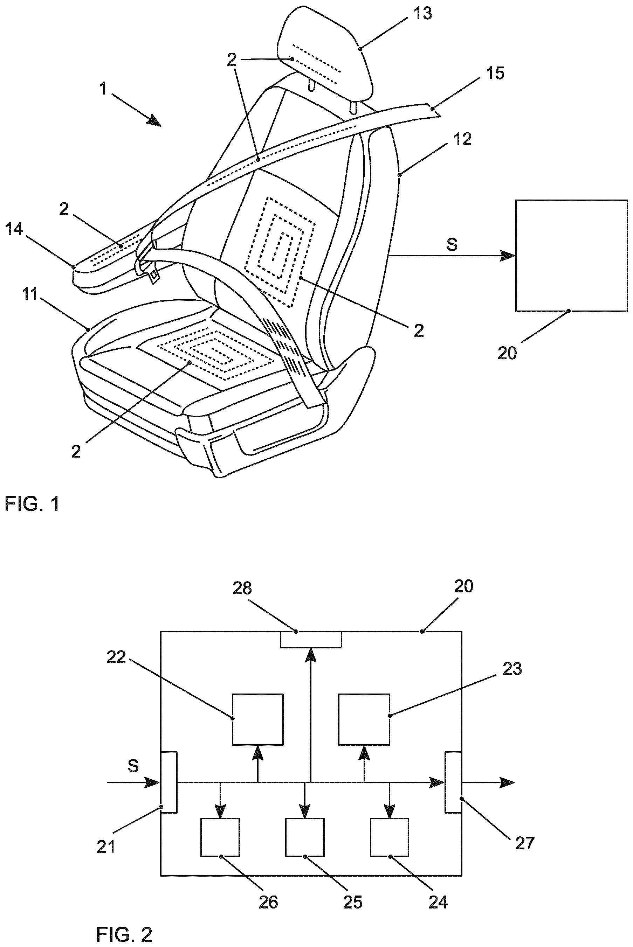

Fig. 1 zeigt schematisch ein System zum Erfassen von Biosignalen einer Person. Das System umfasst eine biometrische Sitzvorrichtung 1. Die biometrische Sitzvorrichtung 1, in diesem Fall ein Sitz für ein Kraftfahrzeug, weist eine Sitzfläche 11 sowie eine Rückenlehne 12 mit einer Kopfstütze 13 auf. Zudem umfasst die biometrische Sitzvorrichtung 1 eine Armlehne 14 und einen Sicherheitsgurt 15. Je nach Verwendungszweck der biometrischen Sitzvorrichtung 1 können einige dieser Elemente entfallen. Beispielsweise ist bei Sitzen in einem Zug üblicherweise kein Sicherheitsgurt 15 vorhanden, während bei Sitzen in einem Fahrgeschäft in der Regel keine Armlehne 14 vorgesehen ist. Die biometrische Sitzvorrichtung 1 weist zumindest ein optisches Sensorelement 2 auf. Im dargestellten Beispiel sind sowohl in der Sitzfläche 11 als auch in der Rückenlehne 12, der Kopfstütze 13, der Armlehne 14 und dem Sicherheitsgurt 15 optische Sensorelemente 2 angeordnet. Das System umfasst zudem eine Auswerteeinheit 20 zum Auswerten von Signalen S des zumindest einen optischen Sensorelementes 2. Die Signale S der verschiedenen optischen Sensorelemente 2 können separat zur Bewertung herangezogen werden, die optischen Sensorelemente 2 können aber auch als ein Messcluster betrachtet werden, d.h. in einer Gesamtbewertung berücksichtigt werden. Das System ist eingerichtet, auf Grundlage der Signale S des zumindest einen optischen Sensorelementes 2 Biosignale einer Person, die sich in bzw. auf der Sitzvorrichtung 1 befindet, zu ermitteln. Aus den Biosignalen ermittelt die Auswerteeinheit 20 Informationen zum Zustand der Person, z.B. zu zumindest einem der folgenden Merkmale: Temperatur, Herzschlag, Herzfrequenzvariabilität, Anpressdruck, Blutdruck, Schweißbildung, Masse der Person, Massenverteilung auf einer Sitzfläche der biometrischen Sitzvorrichtung. Zudem kann die Auswerteeinheit 20 eingerichtet sein, weitere Daten zu empfangen, z.B. Informationen zu einer Verkehrssituation, zu einem Fahrverhalten oder zu Wetterumständen. Auf Basis der verfügbaren Informationen kann die Auswerteeinheit 20 ein Phonokardiogramm erstellen oder eine Zustandsanalyse durchführen, z.B. eine Kardioanalyse, eine Bestimmung einer Stimmungslage oder eine psychophysische Zustandsüberwachung. Von der Auswerteeinheit 20 ermittelte Ergebnisse können der beobachteten Person oder einer anderen berechtigten Person über eine Mensch-Maschine-Schnittstelle (nicht dargestellt) mitgeteilt werden. Zudem kann vorgesehen sein, dass die Auswerteeinheit 20 insbesondere im Falle von Gefahrensituationen Sicherheitsmanöver einleitet.

Fig. 2 zeigt schematisch eine erste Ausführungsform einer Auswerteeinheit 20, die zur Verwendung im System aus Fig. 1 geeignet ist. Die Auswerteeinheit 20 hat einen Eingang 21 zum Empfangen von Signalen S einer biometrischen Sitzvorrichtung. Zudem können optional über den Eingang 21 weitere Informationen empfangen werden, z.B. Informationen zu einer Verkehrssituation, zu einem Fahrverhalten oder zu Wetterumständen. Die Auswerteeinheit 20 hat zudem eine Auswerte-Logik 22 zum Ermitteln von Biosignalen einer Person, die sich in bzw. auf der Sitzvorrichtung befindet, auf Grundlage der empfangenen Signale S. Eine Analyseeinheit 23 dient zum Ermitteln von Informationen zum Zustand der Person aus den Biosignalen. Die Analyseeinheit 23 kann dabei Informationen zu zumindest einem der folgenden Merkmale ermitteln: Temperatur, Herzschlag, Herzfrequenzvariabilität, Anpressdruck, Blutdruck, Schweißbildung, Masse der Person, Massenverteilung auf einer Sitzfläche der biometrischen Sitzvorrichtung. Weiterhin kann die Analyseeinheit 23 auf Basis der Biosignale sowie der weiteren verfügbaren Informationen ein Phonokardiogramm erstellen oder eine Zustandsanalyse vornehmen, z.B. eine Kardioanalyse, eine Bestimmung einer Stimmungslage oder eine psychophysische Zustandsüberwachung. Eine Steuerungseinheit 24 ermöglicht es, die beobachtete Person oder eine andere berechtigte Person mittels einer Benutzerschnittstelle 28 über die von der Analyseeinheit 23 ermittelten Ergebnisse zu informieren. Zudem kann die Steuerungseinheit 24 eingerichtet sein, im Falle von Notsituationen Sicherheitsmanöver einzuleiten. Zu diesem Zweck kann die Steuerungseinheit 24 entsprechende Steuerbefehle über einen Ausgang 27 der Vorrichtung 20 ausgeben.

Die Auswerte-Logik 22, die Analyseeinheit 23 und die Steuerungseinheit 24 können von einer Kontrolleinheit 25 gesteuert werden. Über die Benutzerschnittstelle 28 können gegebenenfalls Einstellungen der Auswerte-Logik 22, der Analyseeinheit 23, der Steuerungseinheit 24 oder der Kontrolleinheit 25 geändert werden. Die in der Vorrichtung 20 anfallenden Daten können bei Bedarf in einem Speicher 26 der Vorrichtung 20 abgelegt werden, beispielsweise für eine spätere Auswertung oder für eine Nutzung durch die Komponenten der Vorrichtung 20. Die Auswerte-Logik 22, die Analyseeinheit 23, die Steuerungseinheit 24 sowie die Kontrolleinheit 25 können als dedizierte Hardware realisiert sein, beispielsweise als integrierte Schaltungen. Natürlich können sie aber auch teilweise oder vollständig kombiniert oder als Software implementiert werden, die auf einem geeigneten Prozessor läuft, beispielsweise auf einer GPU oder einer CPU. Der Eingang 21 und der Ausgang 27 können als getrennte Schnittstellen oder als eine kombinierte bidirektionale Schnittstelle implementiert sein.

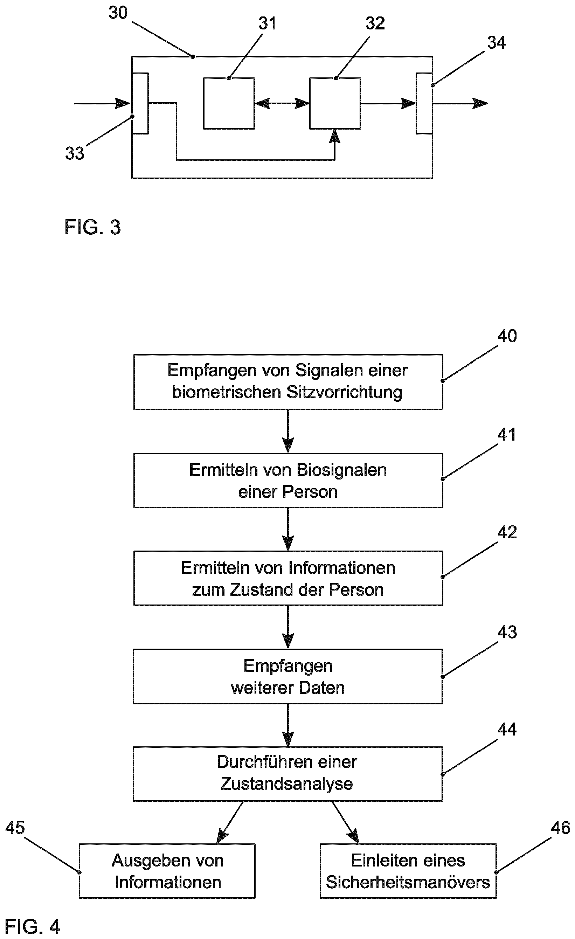

Fig. 3 zeigt schematisch eine zweite Ausführungsform einer Auswerteeinheit 30, die zur Verwendung im System aus Fig. 1 geeignet ist. Die Auswerteeinheit 30 weist einen Prozessor 32 und einen Speicher 31 auf. Beispielsweise handelt es sich bei der Auswerteeinheit 30 um einen Computer oder ein Steuergerät. Im Speicher 31 sind Instruktionen abgelegt, die die Auswerteeinheit 30 bei Ausführung durch den Prozessor 32 veranlassen, die Schritte gemäß dem weiter unten beschriebenen Verfahren auszuführen. Die im Speicher 31 abgelegten Instruktionen verkörpern somit ein durch den Prozessor 32 ausführbares Programm, welches das beschriebene Verfahren realisiert. Die Auswerteeinheit 30 hat einen Eingang 33 zum Empfangen von Informationen, insbesondere von Signalen des optischen Sensorelementes der biometrischen Sitzvorrichtung. Vom Prozessor 32 generierte Daten werden über einen Ausgang 34 bereitgestellt. Darüber hinaus können sie im Speicher 31 abgelegt werden. Der Eingang 33 und der Ausgang 34 können zu einer bidirektionalen Schnittstelle zusammengefasst sein.

Der Prozessor 32 kann eine oder mehrere Prozessoreinheiten umfassen, beispielsweise Mikroprozessoren, digitale Signalprozessoren oder Kombinationen daraus.

Die Speicher 26, 31 der beschriebenen Ausführungsformen können sowohl volatile als auch nichtvolatile Speicherbereiche aufweisen und unterschiedlichste Speichergeräte und Speichermedien umfassen, beispielsweise Festplatten, optische Speichermedien oder Halbleiterspeicher.

Fig. 4 zeigt schematisch ein Verfahren zum Auswerten von Signalen eines optischen Sensors einer biometrischen Sitzvorrichtung. In einem ersten Schritt 40 werden Signale einer biometrischen Sitzvorrichtung empfangen. Aus den empfangenen Signalen werden Biosignale einer Person, die sich in bzw. auf der Sitzvorrichtung befindet, ermittelt 41. Auf Basis der Biosignale werden anschließend Informationen zum Zustand der Person ermittelt 42. Dabei können Informationen zu zumindest einem der folgenden Merkmale ermittelt werden: Temperatur, Herzschlag, Herzfrequenzvariabilität, Anpressdruck, Blutdruck, Schweißbildung, Masse der Person, Massenverteilung auf einer Sitzfläche der biometrischen Sitzvorrichtung. Optional können weitere Daten empfangen werden 43, z.B. Informationen zu einer Verkehrssituation, zu einem Fahrverhalten oder zu Wetterumständen. Auf Basis der verfügbaren Informationen kann ein Phonokardiogramm erstellt oder eine Zustandsanalyse durchgeführt werden 44, z.B. eine Kardioanalyse, eine Bestimmung einer Stimmungslage oder eine psychophysische Zustandsüberwachung. Ergebnisse der Zustandsanalyse oder die Informationen zum Zustand können daraufhin an die beobachtete Person oder eine andere berechtigte Person ausgegeben werden 45. Alternativ oder zusätzlich können im Falle von Notsituationen Sicherheitsmanöver eingeleitet werden 46.

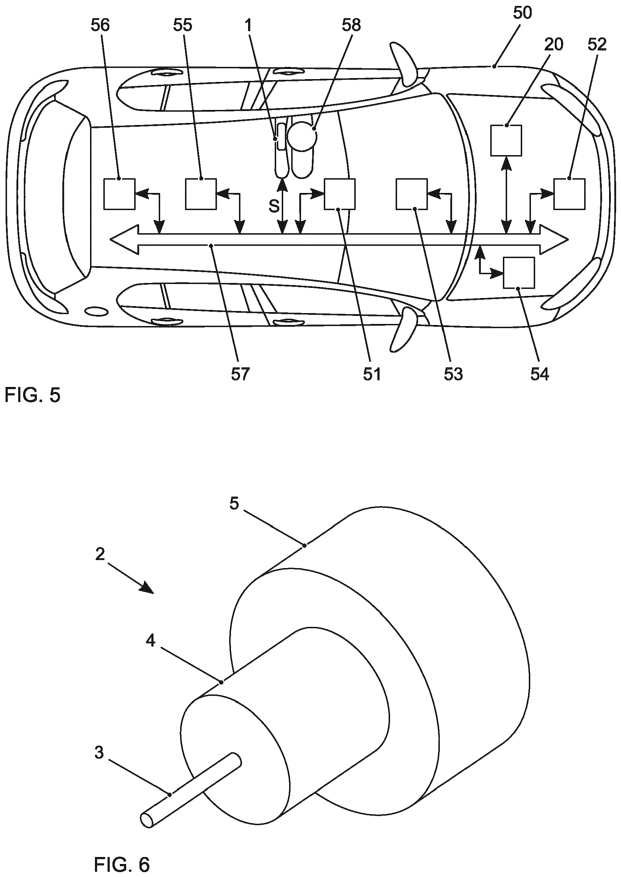

Fig. 5 stellt schematisch ein Fortbewegungsmittel 50 dar, in dem ein erfindungsgemäßes System realisiert ist. In diesem Beispiel handelt es sich beim Fortbewegungsmittel 50 um ein Kraftfahrzeug. Das Kraftfahrzeug weist eine biometrisches Sitzvorrichtung 1 sowie eine Auswerteeinheit 20 zum Auswerten von Signalen eines optischen Sensorelementes der biometrischen Sitzvorrichtung 1 auf. Aus den Biosignalen S einer Person 58, z.B. des Fahrers oder eines anderen Insassen, ermittelt die Auswerteeinheit 20 Informationen zu zumindest einem der folgenden Merkmale: Temperatur, Herzschlag, Herzfrequenzvariabilität, Anpressdruck, Blutdruck, Schweißbildung, Masse der Person 58, Massenverteilung auf einer Sitzfläche der biometrischen Sitzvorrichtung. Weitere Komponenten des Kraftfahrzeugs sind eine Umgebungssensorik 51, z.B. eine Kamera oder ein Radarsensor zur Umfeldüberwachung, ein Regen- oder Nebelsensor, etc., sowie Steuergeräte 52, die Informationen zum Fahrverhalten liefern können, z.B. zum Lenkwinkel, zur Stellung des Gaspedals oder zur Betätigung der Bremse. Auf Basis der verfügbaren Informationen kann die Auswerteeinheit 20 ein Phonokardiogramm erstellen oder eine Kardioanalyse, eine Bestimmung einer Stimmungslage oder eine psychophysische Zustandsüberwachung vornehmen. Von der Auswerteeinheit 20 ermittelte Ergebnisse können der beobachteten Person 58 oder einer anderen berechtigten Person über eine Mensch-Maschine-Schnittstelle 53 mitgeteilt werden. Zudem kann vorgesehen sein, dass insbesondere im Falle von Gefahrensituationen Sicherheitsmanöver eingeleitet werden. Zu diesem Zweck kann die Auswerteeinheit 20 entsprechende Anweisungen an Assistenzsysteme 54 des Kraftfahrzeugs übermitteln. Mittels einer Datenübertragungseinheit 55 kann zudem eine Verbindung zu einem Dienstanbieter aufgebaut werden, beispielsweise zum Übermitteln der am Fahrer gemessenen Daten für eine medizinische Auswertung. Zur Speicherung von Daten ist ein Speicher 56 vorhanden. Der Datenaustausch zwischen den verschiedenen Komponenten des Kraftfahrzeugs 50 erfolgt über ein Netzwerk 57.

Fig. 6 stellt schematisch den Aufbau eines faseroptischen Sensorelementes 2 dar. Dieses hat im Kern einen Lichtwellenleiter 3, z.B. eine Monomodefaser mit einem Durchmesser von 4 µm bis 10 µm. Die Monomodefaser ist von einem Mantel 4 ("Cladding") umschlossen, der beispielsweise einen Durchmesser von 125 µm hat. Auf dem Mantel 4 ist eine Beschichtung 5 ("Coating") aufgebracht, die als mechanischer Schutz für den Mantel 4 dient. Das Coating kann einen Durchmesser von ca. 250 µm haben. In Fig. 6 hat das faseroptische Sensorelement 2 einen kreisförmigen Querschnitt. Es kann alternativ auch einen viereckigen Querschnitt aufweisen. Für die Anfertigung des faseroptischen Sensorelementes 2 stehen unterschiedliche Materialen zur Verfügung, z.B. Glas oder Glasmischungen, Quarz, Elastomere oder Thermoplaste.

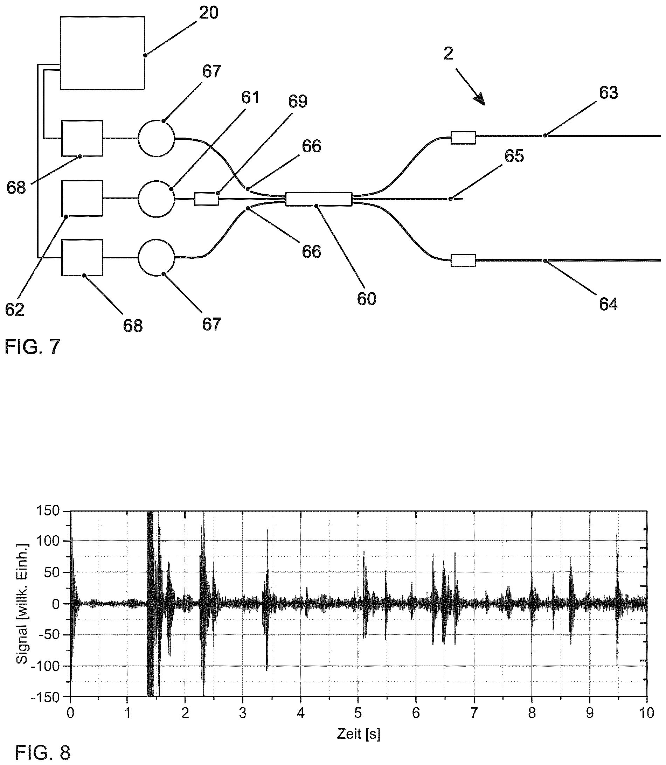

Fig. 7 illustriert beispielhaft das Messprinzip, auf dem das faseroptische Sensorelement 2 basiert. Grundlage für die Messungen ist die faseroptische Interferometrie, für die ein vollständig mittels Faseroptik realisiertes Michelson-Interferometer genutzt wird. Strahlteilung und Strahlkombination werden dabei durch einen faseroptischen 3x3 Single-Mode-Koppler 60 realisiert. Der mittlere Arm des Kopplers 60 ist mit einer Lichtquelle 61 verbunden, z.B. einer VCSEL-Diode (VCSEL: Vertical Cavity Surface Emitting Laser; oberflächenemittierender Laser mit vertikaler Kavität), die eine Wellenlänge von 1310 nm abgibt. Diese Laserdiode wird mit einem Gleichstrom von 3 mA betrieben, der von einem Profiltreiber 62 geliefert wird. Die Strahlung läuft durch den Koppler 60 und teilt sich auf drei Ausgangs-Arme auf, den Sensorarm 63, den Referenzarm 64 und den inaktiven mittleren Arm 65. Die Funktion des Sensorarms 63 und des Referenzarms 64 ist identisch. Der Faseraufbau besteht aus 9 µm Kern, 125 µm Cladding und 250 µm Coating.

Der optische Strahl reflektiert an den Faserenden und läuft durch den Faserkoppler 60 wieder zurück, interferiert dabei und wird in zwei Eingangsarme 66 aufgespalten, wo eine Detektion mittels zweier Photodioden 67 erfolgt. Durch einen optischen Isolator 69 wird dabei eine Rückkopplung mit der Lichtquelle 61 vermieden. Die Signale der Photodioden 67 tragen nun die Information über z.B. Herzschlag, Druck oder Ereignisse, die an einer mit dem faseroptischen Sensorelement 2 ausgerüsteten Sitzvorrichtung verursacht werden. Die Signale werden jeweils mit einem Low-Noise-Transimpedanzverstärker 68 angehoben und anschließend in digitalisierter Form der Auswerteeinheit 20 zugeführt.

Fig. 8 zeigt ein mit dem faseroptischen Sensorelement gemessenes Phonokardiogramm. Zu sehen ist eine akustische ungefilterte Signalaufnahme des Herzschlagverlaufs über eine Messzeit von ca. 10 s, während ein Sicherheitsgurt mit dem faseroptischen Sensorelement am Körper einer Person anliegt. Auf Basis des Phonokardiogramms kann eine Kardioanalyse durchgeführt werden. Analysiert werden kann das Phonokardiogramm unter anderem in Hinblick auf die Frequenz bzw. Anzeichen von Tachykardie (Herzrasen), Bradykardie (verlangsamter Herzschlag) oder Infarkt.

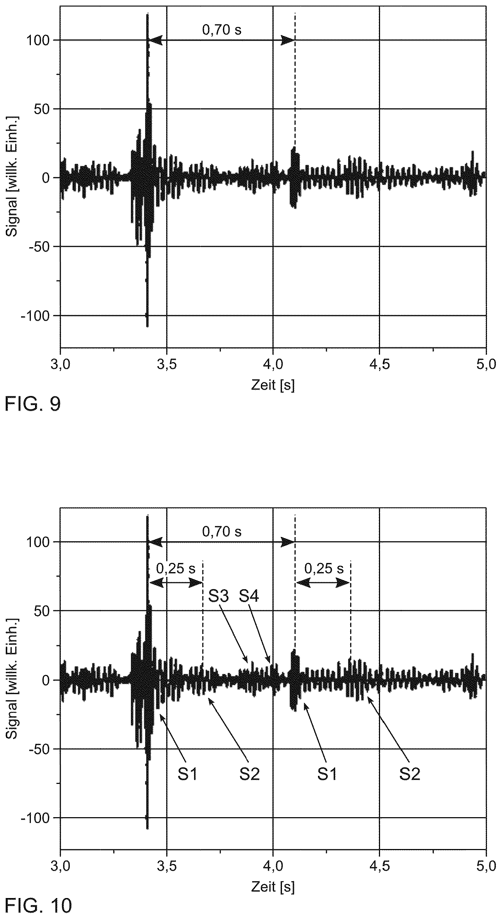

Fig. 9 zeigt ein mit dem faseroptischen Sensorelement gemessenes Herzschlagsignal. Das Signal wurde dabei einer Filterung unterzogen. Nach ca. 5 s liefert der Sensor Information über den Puls (85 Schläge/min) und die Herzfrequenz (1,4 Hz).

Fig. 10 zeigt eine Analyse der Herztöne auf Basis des Herzschlagsignals aus Fig. 9. Das Herzschlagsignal kann genutzt werden, um den einzelnen Herzschlag genauer zu analysieren, d.h. eine Kardioanalyse durchzuführen. Es ist ersichtlich, dass zwei Herztöne (S1 und S2) oder Herzgeräusche auffällig sind, die als normal bzw. anomal klassifiziert werden können. Schwach zu erkennen sind zwei weitere Herztöne (S3 und S4), die weitere Informationen über den Herzzustand der Person liefern können. Auf Grundlage der Kardioanalyse kann beispielsweise ein Vorhofflimmern erkannt werden.

Mit Hilfe von faseroptischen Sensorelementen ist zudem eine Analyse der Hautfeuchtigkeit durch Bestimmung des Hautleitwertes sowie eine Untersuchung des Hand- oder Körperschweißes möglich. Dabei können insbesondere die Elektrolytkonzentration (Na⁺, K⁺, Ca₂⁺, Cl⁻) und der pH-Wert bestimmt werden. Zudem sind Messungen in Bezug auf Glukose, Lactat und Stoffwechselparameter möglich. Für diese Messungen können beispielsweise auf Fluoreszenz basierende Messverfahren genutzt. Ebenso ist es möglich, die optische Faser so zu präparieren, dass entlang der Länge des Sensors kleine Spalte eingearbeitet werden. In die Spalte können die zu messenden Ionen eindringen und dort gelagert werden. Durch ihre Anwesenheit ändern sich Charakteristika des interferometrischen Signals, die ihrerseits gemessen werden können.

### Bezugszeichenliste

- 1: Sitzvorrichtung
- 2: Optisches Sensorelement
- 3: Lichtwellenleiter
- 4: Mantel
- 5: Beschichtung
- 11: Sitzfläche
- 12: Rückenlehne
- 13: Kopfstütze
- 14: Armlehne
- 15: Sicherheitsgurt
- 20: Auswerteeinheit
- 21: Eingang
- 22: Auswerte-Logik
- 23: Analyseeinheit
- 24: Steuerungseinheit
- 25: Kontrolleinheit
- 26: Speicher
- 27: Ausgang
- 28: Benutzerschnittstelle
- 30: Auswerteeinheit
- 31: Speicher
- 32: Prozessor
- 33: Eingang
- 34: Ausgang
- 40: Empfangen von Signalen einer biometrischen Sitzvorrichtung
- 41: Ermitteln von Biosignalen einer Person
- 42: Ermitteln von Informationen zum Zustand der Person
- 43: Empfangen weiterer Daten
- 44: Erstellen eines Phonokardiogramms oder Durchführen einer Zustandsanalyse
- 45: Ausgeben von Informationen
- 46: Einleiten eines Sicherheitsmanövers
- 50: Kraftfahrzeug
- 51: Umgebungssensorik
- 52: Steuergerät
- 53: Mensch-Maschine-Schnittstelle
- 54: Assistenzsystem
- 55: Datenübertragungseinheit
- 56: Speicher
- 57: Netzwerk
- 58: Person
- 60: Koppler
- 61: Lichtquelle
- 62: Profiltreiber
- 63: Sensorarm
- 64: Referenzarm
- 65: Inaktiver Arm
- 66: Eingangsarm
- 67: Photodiode
- 68: Transimpedanzverstärker
- 69: Optischer Isolator
- S: Signal
- S1, S2, S3, S4: Herzton

## Patentansprüche

1. System zum Erfassen von Biosignalen einer Person (58), mit:
- einer biometrischen Sitzvorrichtung (1) für die Person (58), wobei die biometrische Sitzvorrichtung (1) zumindest ein auf optischer Interferometrie basierendes faseroptisches Sensorelement (2) aufweist, wobei das faseroptische Sensorelement (2) als Michelson-Interferometer ausgestaltetet ist und eine Lichtquelle (61) und einen Faserkoppler (60) aufweist, und wobei das Licht der Lichtquelle (61) durch den Faserkoppler (60) in einen Sensorarm (63) und einen Referenzarm (64) eingekoppelt wird, das eingekoppelte Licht an den Faserenden reflektiert wird, durch den Faserkoppler (60) wieder zurückläuft und dabei interferiert; und
- einer Auswerteeinheit (20) zum Auswerten von Signalen (S) des zumindest einen faseroptischen Sensorelementes (2);
wobei das System eingerichtet ist, auf Grundlage der Signale (S) des zumindest einen faseroptischen Sensorelementes (2) Biosignale der Person (58) zu ermitteln;
**dadurch gekennzeichnet, dass** das faseroptische Sensorelement (2) zwei Photodioden (67) aufweist, die Lichtquelle (61) mit einem mittleren Arm des Faserkopplers (60) verbunden ist, der mit einem optischen Isolator (69) versehen ist, und das an den Faserenden reflektierte und im Faserkoppler (60) interferierende Licht vom Faserkoppler (60) in zwei Eingangsarme (66) aufgespalten wird, wo eine Detektion mittels der zwei Photodioden (67) erfolgt.

2. System gemäß Anspruch 1, wobei das faseroptische Sensorelement zumindest eine Monomodefaser aufweist.

3. System gemäß Anspruch 1 oder 2, wobei die Auswerteeinheit (20) eingerichtet ist, eine durch eine Kraft hervorgerufene Änderung zumindest einer optischen Eigenschaft des zumindest einen faseroptischen Sensorelementes (2) zu erfassen.

4. System gemäß einem der vorherigen Ansprüche, wobei das zumindest eine faseroptische Sensorelement (2) in einer Sitzfläche (11), einer Rückenlehne (12), einer Kopfstütze (13) oder einer Armlehne (14) der biometrischen Sitzvorrichtung (1) angeordnet ist.

5. System gemäß einem der vorherigen Ansprüche, wobei das zumindest eine faseroptische Sensorelement (2) in einem Sicherheitsgurt (15) oder einem Sicherheitsbügel der biometrischen Sitzvorrichtung (1) angeordnet ist.

6. System gemäß einem der vorherigen Ansprüche, wobei die Auswerteeinheit (20) eingerichtet ist, aus den Signalen (S) des zumindest einen faseroptischen Sensorelementes (2) Informationen zu zumindest einem der folgenden Merkmale zu ermitteln: Temperatur, Herzschlag, Herzfrequenzvariabilität, Anpressdruck, Blutdruck, Schweißbildung, Masse der Person (58), Massenverteilung auf einer Sitzfläche (11) der biometrischen Sitzvorrichtung (1).

7. System gemäß Anspruch 6, wobei die Auswerteeinheit (20) eingerichtet ist, auf Basis der ermittelten Informationen ein Phonokardiogramm zu erstellen oder eine Kardioanalyse, eine Bestimmung einer Stimmungslage oder eine psychophysische Zustandsüberwachung vorzunehmen.

8. Fortbewegungsmittel (50), **dadurch gekennzeichnet, dass** das Fortbewegungsmittel (50) ein System gemäß einem der Ansprüche 1 bis 7 zum Erfassen von Biosignalen eines Insassen des Fortbewegungsmittels (50) aufweist.

## Claims

1. A system for detecting biosignals of a person (58), comprising:
- a biometric seat device (1) for the person (58), the biometric seat device (1) comprising at least one fiber-optic sensor element (2) based on optical interferometry, the fiber-optic sensor element (2) being designed as a Michelson interferometer and comprising a light source (61) and a fiber coupler (60), and the light of the light source (61) being coupled through the fiber coupler (60) into a sensor arm (63) and a reference arm (64), the coupled light being reflected at the fiber ends, returning through the fiber coupler (60) and thereby interfering; and
- an evaluation unit (20) for evaluating signals (S) from the at least one fiber-optic sensor element (2);
the system being configured to determine biosignals of the person (58) on the basis of the signals (S) from the at least one fiber-optic sensor element (2);
**characterized in that** the fiber-optic sensor element (2) comprises two photodiodes (67), the light source (61) is connected to a central arm of the fiber coupler (60), which is provided with an optical insulator (69), and the light reflected at the fiber ends and interfering in the fiber coupler (60) is split by the fiber coupler (60) into two input arms (66), where detection takes place by means of the two photodiodes (67).

2. The system according to claim 1, wherein the fiber-optic sensor element comprises at least one single-mode fiber.

3. The system according to either claim 1 or claim 2, wherein the evaluation unit (20) is configured to detect a change in at least one optical property of the at least one fiber-optic sensor element (2) caused by a force.

4. The system according to any of the previous claims, wherein the at least one fiber-optic sensor element (2) is arranged in a seat surface (11), a backrest (12), a headrest (13) or an armrest (14) of the biometric seat device (1).

5. The system according to any of the preceding claims, wherein the at least one fiber-optic sensor element (2) is arranged in a seat belt (15) or a safety bar of the biometric seat device (1).

6. The system according to any of the previous claims, wherein the evaluation unit (20) is configured to determine, from the signals (S) of the at least one fiber-optic sensor element (2), information relating to at least one of the following features: temperature, heartbeat, heart rate variability, contact pressure, blood pressure, perspiration, mass of the person (58), mass distribution on a seat surface (11) of the biometric seat device (1).

7. The system according to claim 6, wherein the evaluation unit (20) is configured to create a phonocardiogram or to carry out a cardioanalysis, a determination of mood or psychophysical state monitoring on the basis of the determined information.

8. A transportation means (50), **characterized in that** the transportation means (50) comprises a system according to any of claims 1 to 7 for detecting biosignals of an occupant of the transportation means (50).

## Revendications

1. Système pour la détection de signaux biologiques d'une personne (58), comportant :
- un dispositif d'assise biométrique (1) pour la personne (58), dans lequel le dispositif d'assise biométrique (1) présente au moins un élément de capteur à fibre optique (2) basé sur l'interférométrie optique, dans lequel l'élément de capteur à fibre optique (2) est réalisé comme un interféromètre de Michelson et présente une source de lumière (61) et un coupleur de fibres (60), et dans lequel la lumière de la source de lumière (61) est couplée par le coupleur de fibres (60) dans un bras de capteur (63) et un bras de référence (64), la lumière couplée est réfléchie au niveau des extrémités des fibres, revient à travers le coupleur de fibres (60) et interfère ainsi ; et
- une unité d'évaluation (20) pour l'évaluation de signaux (S) de l'au moins un élément de capteur à fibre optique (2) ;
dans lequel le système est conçu pour déterminer des signaux biologiques de la personne (58) sur la base des signaux (S) du au moins un élément de capteur à fibre optique (2) ;
**caractérisé en ce que** l'élément de capteur à fibre optique (2) présente deux photodiodes (67), la source de lumière (61) est reliée à un bras central du coupleur de fibres (60) qui est pourvu d'un isolateur optique (69), et la lumière réfléchie au niveau des extrémités des fibres et interférant dans le coupleur de fibres (60) est divisée par le coupleur de fibres (60) en deux bras d'entrée (66) où une détection est effectuée au moyen des deux photodiodes (67).

2. Système selon la revendication 1, dans lequel l'élément de capteur à fibre optique présente au moins une fibre monomode.

3. Système selon la revendication 1 ou 2, dans lequel l'unité d'évaluation (20) est conçue pour détecter une modification, provoquée par une force, d'au moins une propriété optique de l'au moins un élément de capteur à fibre optique (2).

4. Système selon l'une des revendications précédentes, dans lequel l'au moins un élément de capteur à fibre optique (2) est disposé dans une assise (11), un dossier (12), un appui-tête (13) ou un accoudoir (14) du dispositif d'assise biométrique (1).

5. Système selon l'une des revendications précédentes, dans lequel l'au moins un élément de capteur à fibre optique (2) est disposé dans une ceinture de sécurité (15) ou un arceau de sécurité du dispositif d'assise biométrique (1).

6. Système selon l'une des revendications précédentes, dans lequel l'unité d'évaluation (20) est conçue pour déterminer, à partir des signaux (S) de l'au moins un élément de capteur à fibre optique (2), des informations concernant au moins l'une des caractéristiques suivantes : température, rythme cardiaque, variabilité de la fréquence cardiaque, pression d'appui, pression sanguine, transpiration, masse de la personne (58), répartition de la masse sur une surface d'assise (11) du dispositif d'assise biométrique (1).

7. Système selon la revendication 6, dans lequel l'unité d'évaluation (20) est conçue, sur la base des informations déterminées, pour établir un phonocardiogramme ou pour procéder à une analyse cardiaque, à une détermination d'un état d'humeur ou à une surveillance d'état psychophysique.

8. Moyen de transport (50), **caractérisé en ce que** le moyen de transport (50) présente un système selon l'une des revendications 1 à 7 pour la détection de signaux biologiques d'un occupant du moyen de transport (50).
